# EUROPÄISCHE PATENTSCHRIFT

(11) **EP 3 738 993 B1**
(45) Veröffentlichungstag und Bekanntmachung des Hinweises auf die Patenterteilung: **15.09.2021**
(21) Anmeldenummer: 20172692.4
(22) Anmeldetag: 04.05.2020
(51) Int. Cl.: C08G 77/388, C08L 83/08, A01N 37/18, A61Q 17/02

(54) **VERZÖGERTE FREISETZUNG EINER INSEKTEN ABWEHRENDEN VERBINDUNG**
SLOW RELEASE OF INSECT REPELLENT COMPOUND
LIBÉRATION RETARDÉE D'UN COMPOSÉ INSECTIFUGE

(30) Priorität: 10.05.2019 DE 102019112267
(43) Veröffentlichungstag der Anmeldung: 18.11.2020
(73) Patentinhaber: Henkel AG & Co. KGaA, 40589 Düsseldorf (DE)
(72) Erfinder: SCHAEFER, Sascha, 40822 Mettmann (DE); SAUF, Silvia, 42555 Velbert (DE); KROPF, Christian, 40724 Hilden (DE)

(56) Entgegenhaltungen:
- EP-A1- 3 175 865
- WO-A2-2007/093297

## Beschreibung

### GEBIET DER ERFINDUNG

Die Erfindung betrifft spezifische Kieselsäureester-Icaridin-Verbindungen der Formeln (I) und (II) und deren Herstellung. Ferner betrifft die Erfindung ein Insektenabwehrmittel, dass mindestens eine Verbindung der Formel (I) und/oder Formel (II) enthält sowie die Verwendung der Verbindung der Formel (I) oder der Formel (II) zur Freisetzung von Icaridin.

### HINTERGRUND DER ERFINDUNG

Auf dem Gebiet der Insektenabwehrmittel gilt es einen möglichst langanhaltenden und ausreichenden Schutz gegen Insekten aller Art insbesondere stechende und blutsaugende Vertreter zu gewährleisten und dabei ein Maximum an Komfort zu ermöglichen. Letzteres wird dadurch beeinträchtigt, dass wiederholtes Applizieren des Insektenabwehrmittels nötig ist, um den Schutz aufrecht zu erhalten, da sich die gängigen Wirkstoffe wie Icaridin schnell verflüchtigen. Die Aufgabe der vorliegenden Erfindung war folglich das Bereitstellen einer Verbindung, welche Icaridin bereitstellen kann, jedoch weniger flüchtig und stabiler ist.

Die Erfinder der vorliegenden Erfindung haben gefunden, dass dies durch die spezifischen Kieselsäureester-Icaridin-Verbindungen der vorliegenden Erfindung möglich ist. Dabei wurde überraschend gefunden, dass durch die Verwendung der spezifischen Verbindungen eine kontrollierte Freisetzung über Zeit bei gleichzeitiger ausreichender Stabilität gegen Hydrolyse, so dass auch wässrige Formulierungen eingesetzt werden können, möglich ist. Dadurch ist eine geringere und somit Ressourcen schonendere Dosierung des Wirkstoffs, eine kontrollierte Freisetzung über einen längeren Zeitraum und eine verbesserte Lagerstabilität in Insektenabwehrmitteln möglich.

WO2007/093297 A2 offenbart Arthropoden- und Plathelminthen-Abwehrmittel auf Basis von Piperidin-Derivaten in Wasser-in-ÖI-Formulierungen. US2017/225958 A1 offenbart die Herstellung von funktionellen Partikeln aufweisend Wirkstoffe und Silan-basierende Verbindungen.

### ZUSAMMENFASSUNG DER ERFINDUNG

Deshalb betrifft die Erfindung in einem ersten Aspekt eine Verbindung der Formel (I) 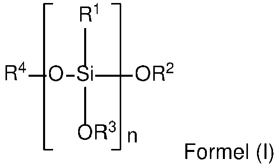 oder der Formel (II) 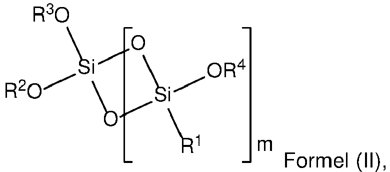 wobei
R¹ für Wasserstoff, eine Hydroxylgruppe, einen Kohlenwasserstoffrest mit 1 bis 6 Kohlenstoffatomen oder OR⁵ steht; und
R² bis R⁵ jeweils unabhängig voneinander für Wasserstoff oder einen Rest mit der Formel (III) 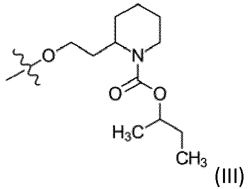 stehen; und
n für eine ganze Zahl von 1 bis 50 steht; und
m für eine ganze Zahl von 1 bis 20 steht,
wobei mindestens einer der Reste R² bis R⁵ 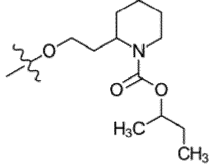 ist.

In einem zweiten Aspekt betrifft die Erfindung ein Insektenabwehrmittel umfassend mindestens eine Verbindung der Formel (I) und/oder Formel (II) gemäß der vorliegenden Erfindung.

In einem dritten Aspekt betrifft die Erfindung die Verwendung der Verbindung nach Formel (I) oder Formel (II) gemäß der vorliegenden Erfindung zur Freisetzung von Icaridin.

Schließlich betrifft die Erfindung in einem vierten Aspekt ein Verfahren zur Herstellung einer Verbindung nach Formel (I) oder Formel (II) gemäß der vorliegenden Erfindung, umfassend:
Umsetzen von Icaridin mit einem Kieselsäureester in der Gegenwart einer Base.

Diese und weitere Ausführungsformen, Merkmale und Vorteile der Erfindung werden für den Fachmann aus dem Studium der folgenden detaillierten Beschreibung und Ansprüche ersichtlich. Dabei können einzelne beschriebene Merkmale oder Ausführungsformen der Erfindung mit anderen Merkmalen oder Ausführungsformen der Erfindung kombiniert werden ohne dass diese im Rahmen der Erfindung in Kombination beschrieben wurden. Es ist selbstverständlich, dass die hierin enthaltenen Beispiele die Erfindung beschreiben und veranschaulichen sollen, diese aber nicht einschränken und insbesondere die Erfindung nicht auf die Beispiele beschränkt ist.

### DETAILLIERTE BESCHREIBUNG DER ERFINDUNG

"Mindestens ein", wie hierin verwendet, bezieht sich auf 1 oder mehr, beispielsweise 2, 3, 4, 5, 6, 7, 8, 9 oder mehr. Im Zusammenhang mit den hierin beschriebenen Verbindungen bezieht sich diese Angabe nicht auf die absolute Menge an Molekülen, sondern auf die Art der Verbindung. "Mindestens eine Verbindung der Formel (I)" bedeutet daher beispielsweise, dass nur eine Art der Verbindungen der Formel (I) oder mehrere verschiedene Arten der Verbindungen der Formel (I), ohne Angaben über die Menge der einzelnen Verbindungen zu machen, enthalten sein können.

Alle im Zusammenhang mit dem hierin beschriebenen Verfahren angegeben Mengenangaben beziehen sich, sofern nichts anderes angegeben ist, auf Gew.-% jeweils bezogen auf das Gesamtgewicht der Zusammensetzung. Des Weiteren beziehen sich derartige Mengenangaben, die sich auf mindestens einen Bestandteil beziehen, immer auf die Gesamtmenge dieser Art von Bestandteil, der in der Zusammensetzung enthalten ist, sofern nicht explizit etwas anderes angegeben ist. Das heißt, dass sich derartige Mengenangaben, beispielsweise im Zusammenhang mit "mindestens einer Verbindung der Formel (I)", auf die Gesamtmenge von Verbindungen der Formel (I), welche in der Zusammensetzung enthalten sind, bezieht, wenn nicht explizit etwas anderes angegeben ist.

Zahlenwerte, die hierin ohne Dezimalstellen angegeben sind, beziehen sich jeweils auf den vollen angegebenen Wert mit einer Dezimalstelle. So steht beispielsweise "99 %" für "99,0 %".

Numerische Bereiche, die in dem Format "in/von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

Ein Aspekt der Erfindung betrifft eine Verbindung der Formel (I) 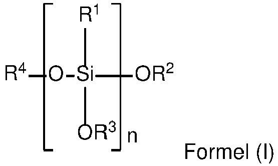 oder der Formel (II) 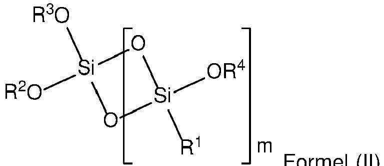 wobei
R¹ für Wasserstoff, eine Hydroxylgruppe, einen Kohlenwasserstoffrest mit 1 bis 6 Kohlenstoffatomen oder OR⁵ steht; und
R² bis R⁵ jeweils unabhängig voneinander für Wasserstoff oder einen Rest mit der Formel (III) 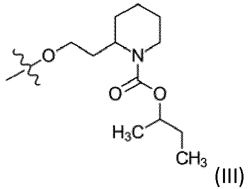 stehen; und
n für eine ganze Zahl von 1 bis 50 steht; und
m für eine ganze Zahl von 1 bis 20 steht,
wobei mindestens einer der Reste R² bis R⁵ 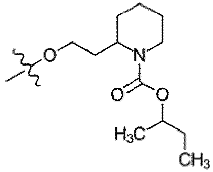 ist.

Dabei umfasst die Formel (III), wenn auch nicht abgebildet, alle möglichen Stereoisomere.

In bevorzugten Ausführungsformen ist R¹ ein Kohlenwasserstoffrest mit 1 bis 6 Kohlenstoffatomen, stärker bevorzugt ein linearer, verzweigter oder cyclischer Alkylrest mit bis zu 6 Kohlenstoffatomen, ein Alkenylrest mit bis zu 6 Kohlenstoffatomen, ein Alkinylrest mit bis zu 6 Kohlenstoffatomen, insbesondere ein linearer oder verzweigter Alkylrest mit bis zu 6 Kohlenstoffatomen. Insbesondere handelt es sich bei R¹ um einen Methyl-, Ethyl-, n-Propyl-, i-Propyl, i-Butyl-, n-Butyl-, t-Butyl-, n-Pentyl-, oder i-Pentylrest, ganz besonders bevorzugt um einen Ethylrest.

Die Oligomerisierungsgrade n der Verbindungen der Formel (I) sind bevorzugt Werte ausgewählt aus dem Bereich von 1 bis 40, stärker bevorzugt 2 bis 20 und noch stärker bevorzugt 3 bis 10, was eine bevorzugte Ausführungsform der Erfindung ist.

Analog dazu sind die Oligomerisierungsgrade m der Verbindungen der Formel (II) bevorzugt Werte ausgewählt aus dem Bereich von 2 bis 15, stärker bevorzugt 2 bis 10 und noch stärker bevorzugt 2 bis 5, was ebenfalls eine bevorzugte Ausführungsform der Erfindung ist.

Da die Ausgangsverbindungen für die Herstellung der Kieselsäureester aus ökonomischen Gründen bevorzugt keine Reinstoffe sind, sondern technische Gemische von Oligokieselsäureestern niederer Alkohole mit unterschiedlichen Oligomerisierungsgraden, findet sich eine Verteilung der Oligomerisierungsgrade auch in den Kieselsäureestern wieder, die dem Ausgangsmaterial entsprechen kann oder durch die Reaktionsbedingungen modifiziert ist.

Die Kieselsäureester-Icaridin-Verbindungen zeichnen sich durch eine gute Hydrolysestabilität aus und können auch in wässrigen Medien oder in Herstellprozessen für Granulate eingesetzt werden, ohne dabei übermäßige Aktivitätsverluste zu erleiden.

Ferner betrifft die vorliegende Erfindung ein Insektenabwehrmittel umfassend mindestens eine Verbindung der Formel (I) und/oder Formel (II) gemäß der vorliegenden Erfindung.

In einer bevorzugten Ausführungsform enthält das Insektenabwehrmittel die mindestens eine Verbindung der Formel (I) und/oder Formel (II) in 0,001 bis 100 Gew.-%, bevorzugt 0,1 bis 50 Gew.-%, stärker bevorzugt 1 bis 30 Gew.-%, bezogen auf das Gesamtgewicht des Insektenabwehrmittels.

Das Insektenabwehrmittel kann in jeder dem Fachmann auf dem Gebiet bekannten Form vorliegen. Bevorzugt ist es ein Spray, eine Creme, eine Paste oder eine Kerze.

Das Insektenabwehrmittel liegt bevorzugt in fester oder flüssiger Form vor, insbesondere in flüssiger Form.

Das Insektenabwehrmittel kann zusätzlich weitere, auf dem Gebiet üblich Additive umfassen. Insbesondere bevorzugt sind diese ausgewählt aus Lösungsmitteln, insbesondere organischen Lösungsmitteln und/oder Wasser, Verdickern, wie Polymeren, von Formel (I) und (II) verschiedenen Insekten abwehrenden Verbindungen, Farbstoffen, Duftstoffen.

Geeignete beispielhafte Insektenabwehrmittel sind beispielsweise in der WO2017094600 A1, EP1738745 A1, EP1762221 A1, WO2018223181 A1 und WO2018039376 A1 offenbart. Dabei kann die mindestens eine erfindungsgemäße Verbindung der Formel (I) und/oder (II) den Wirkstoff in den Zusammensetzungen der vorstehend genannten Druckschriften ersetzten oder zusätzlich zu diesen enthalten sein.

Aus den beschriebenen Verbindungen der Formel (I) und/oder (II) kann Icaridin, bevorzugt durch Hydrolyse, freigesetzt werden.

Des Weiteren betrifft die vorliegende Erfindung die Verwendung der Verbindung nach Formel (I) oder Formel (II) wie in der vorliegenden Erfindung definiert zur Freisetzung von Icaridin, insbesondere als Insekten abwehrende Verbindung.

Schließlich betrifft die vorliegende Erfindung ein Verfahren zur Herstellung einer Verbindung nach Formel (I) oder Formel (II) gemäß der vorliegenden Erfindung, umfassend:
Umsetzen von Icaridin mit einem Kieselsäureester in der Gegenwart einer Base.

Die Kieselsäureester entsprechend dabei den in Formel (I) und Formel (II) definierten Verbindungen, wobei anstelle der Formel (III) ein Wasserstoffrest vorliegt.

In einer bevorzugten Ausführungsform ist die Base NaOH oder KOH, insbesondere KOH.

In einer weiteren bevorzugten Ausführungsform wird die Umsetzung unter Erwärmen, insbesondere auf 85 bis 120 °C, besonders bevorzugt 110 bis 115 °C, durchgeführt.

### BEISPIELE

35,5 g Icaridin wurden in einem 100 mL Rundhalskolben unter Stickstoffatmosphäre vorgelegt und 14,6 g Silan TES 40 (Wacker AG) und 0,02 g KOH Plätzchen zugegeben. Die erhaltene farblose Lösung wurde langsam auf 100°C erwärmt (Heizblock ca. 115 °C). Nach 2,5 h wurde die Lösung abgekühlt und bei Raumtemperatur über Nacht gerührt. Anschließend wurde die Lösung erwärmt und bei 100°C Innentemperatur für weitere 7h gerührt. Nach kurzem Anlegen von Unterdruck und Erwärmen auf 50°C wurden 35,5g einer farblosen, klaren, viskosen Masse erhalten (85,5% Icaridin-Gehalt, ermittelt via GC).

## Patentansprüche

1. Eine Verbindung der Formel (I) 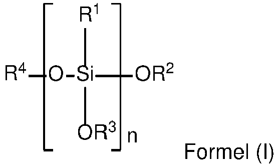 oder der Formel (II) 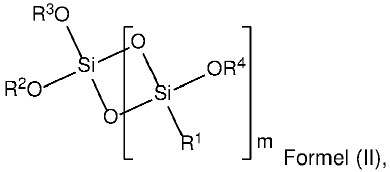 wobei
R¹ für Wasserstoff, eine Hydroxylgruppe, einen Kohlenwasserstoffrest mit 1 bis 6 Kohlenstoffatomen
oder OR⁵ steht; und
R² bis R⁵ jeweils unabhängig voneinander für Wasserstoff oder einen Rest mit der Formel (III) 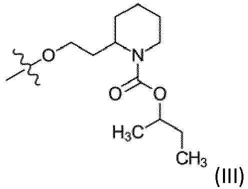 stehen; und
n für eine ganze Zahl von 1 bis 50 steht; und
m für eine ganze Zahl von 1 bis 20 steht,
wobei mindestens einer der Reste R² bis R⁵ 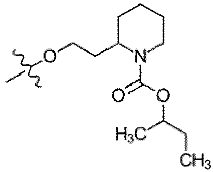 ist.

2. Die Verbindung gemäß Anspruch 1, wobei
R¹ für einen Alkylrest mit 1 bis 6 Kohlenstoffatomen, bevorzugt Methyl oder Ethyl steht.

3. Insektenabwehrmittel umfassend mindestens eine Verbindung der Formel (I) und/oder Formel (II) nach Anspruch 1 oder 2.

4. Insektenabwehrmittel gemäß Anspruch 3, wobei die mindestens eine Verbindung der Formel (I) und/oder Formel (II) in 0,001 bis 100 Gew.-%, bevorzugt 0,1 bis 50 Gew.-%, stärker bevorzugt 1 bis 30 Gew.-%, bezogen auf das Gesamtgewicht des Insektenabwehrmittels enthalten sind.

5. Insektenabwehrmittel gemäß Anspruch 3 oder 4, wobei das Insektenabwehrmittel als Spray, Creme, Paste oder Kerze vorliegt.

6. Insektenabwehrmittel gemäß einem der Ansprüche 3 bis 5, wobei das Insektenabwehrmittel in fester oder flüssiger Form vorliegt.

7. Insektenabwehrmittel gemäß einem der Ansprüche 3 bis 6, ferner enthaltend mindestens eine Verbindung ausgewählt aus Lösungsmitteln, Verdickern, von Formel (I) und (II) verschiedenen Insekten abwehrenden Verbindungen, Farbstoffen und Duftstoffen.

8. Verwendung der Verbindung nach Formel (I) oder Formel (II) wie in Anspruch 1 oder 2 definiert zur Freisetzung von Icaridin, insbesondere als Insekten abwehrende Verbindung.

9. Verfahren zur Herstellung einer Verbindung nach Formel (I) oder Formel (II) wie in Anspruch 1 oder 2 definiert, umfassend:
Umsetzen von Icaridin mit einem Kieselsäureester in der Gegenwart einer Base.

## Claims

1. A compound of formula (I) 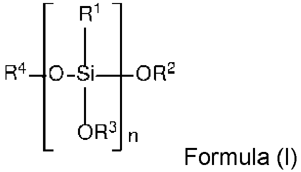 or of formula (II) 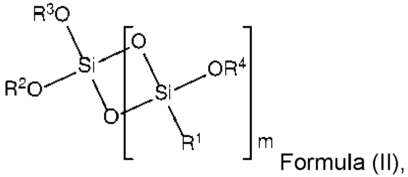 where
R¹ represents hydrogen, a hydroxyl group, a hydrocarbon functional group having 1 to 6 carbon atoms
or OR⁵; and
R² to R⁵ each represent, independently of one another, hydrogen or a functional group of formula (III) 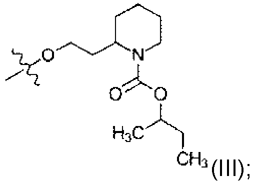 and
n represents an integer from 1 to 50; and
m represents an integer from 1 to 20,
where at least one of the functional groups R² to R⁵ is

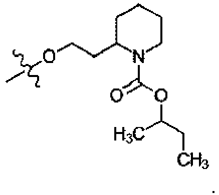

2. The compound according to claim 1 wherein
R¹ represents an alkyl functional group having 1 to 6 carbon atoms, preferably methyl or ethyl.

3. An insect repellent comprising at least one compound of formula (I) and/or formula (II) according to claim 1 or 2.

4. The insect repellent according to claim 3, wherein the at least one compound of formula (I) and/or formula (II) is contained in 0.001 to 100 wt.%, preferably 0.1 to 50 wt.%, more preferably 1 to 30 wt.%, based on the total weight of the insect repellent.

5. The insect repellent according to claim 3 or 4, wherein the insect repellent is in the form of a spray, cream, paste or candle.

6. The insect repellent according to one of claims 3 to 5, wherein the insect repellent is in solid or liquid form.

7. The insect repellent according to one of claims 3 to 6, further containing at least one compound selected from solvents, thickeners, insect repellent compounds other than formula (I) and (II), dyes and fragrances.

8. The use of the compound according to formula (I) or formula (II), as specified in claim 1 or 2, for releasing icaridin, in particular as an insect repellent compound.

9. A method for preparing a compound according to formula (I) or formula (II), as specified in claim 1 or 2, comprising:
reacting icaridin with a silicic acid ester in the presence of a base.

## Revendications

1. Composé de formule (I) 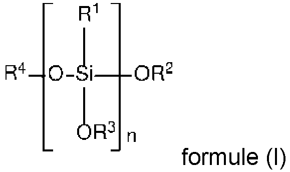 ou de formule (II) 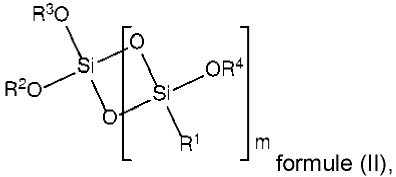 dans laquelle
R¹ représente l'hydrogène, un groupe hydroxyle, un radical hydrocarboné comportant 1 à 6 atomes de carbone
ou OR⁵; et
R² à R⁵ représentent respectivement indépendamment les uns des autres l'hydrogène ou un radical de formule (III) ; 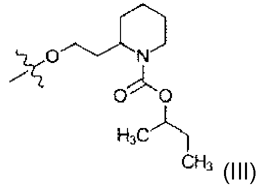 et
n représente un nombre entier compris entre 1 et 50 ; et
m représente un nombre entier compris entre 1 et 20,
dans laquelle au moins un des radicaux R² à R⁵ est

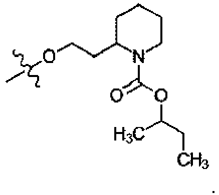

2. Composé selon la revendication 1, dans lequel
R¹ représente un radical alkyle comportant 1 à 6 atomes de carbone, de préférence méthyle ou éthyle.

3. Produit insectifuge comprenant au moins un composé de formule (I) et/ou de formule (II) selon la revendication 1 ou 2.

4. Produit insectifuge selon la revendication 3, dans lequel l'au moins un composé de formule (I) et/ou de formule (II) est contenu dans une quantité de 0,001 à 100 % en poids, de préférence de 0,1 à 50 % en poids, plus préférablement de 1 à 30 % en poids, par rapport au poids total du produit insectifuge.

5. Produit insectifuge selon la revendication 3 ou 4, dans lequel le produit insectifuge est sous la forme d'un spray, d'une crème, d'une pâte ou d'une bougie.

6. Produit insectifuge selon l'une des revendications 3 à 5, dans lequel le produit insectifuge est sous forme solide ou liquide.

7. Produit insectifuge selon l'une des revendications 3 à 6, contenant en outre au moins un composé choisi parmi les solvants, les épaississants, différents composés insectifuges de formules (I) et (II), les colorants et les parfums.

8. Utilisation du composé de formule (I) ou de formule (II) tel que défini à la revendication 1 ou 2 permettant de libérer de l'icaridine, en particulier en tant que composé insectifuge.

9. Procédé permettant de préparer un composé de formule (I) ou de formule (II) tel que défini à la revendication 1 ou 2, comprenant :
le fait de faire réagir de l'icaridine avec un ester d'acide silicique en présence d'une base.
